# EUROPEAN PATENT SPECIFICATION

(11) **EP 4 251 060 B1**
(45) Date of publication and mention of the grant of the patent: **07.10.2026**
(21) Application number: 21830756.9
(22) Date of filing: 19.11.2021
(51) Int. Cl.: A61B 8/00, A61B 8/08, A61B 17/34

(54) **PORTABLE VESSEL SCANNER**
TRAGBARER GEFÄSSSCANNER
SCANNER DE VAISSEAU PORTABLE

(30) Priority: 30.11.2020 IT 202000029084
(43) Date of publication of application: 04.10.2023
(73) Proprietor: Università degli Studi della Campania "Luigi Vanvitelli", 81100 Caserta (CE) (IT)
(72) Inventor: MARFELLA, Raffaele, 81100 Caserta (IT); PAOLISSO, Giuseppe, 81100 Caserta (IT); PAOLISSO, Pasquale, 80138 Napoli (IT); SARDU, Celestino, 81100 Caserta (IT); ZENI, Luigi, 81100 Caserta (IT); VALLIFUOCO, Raffaele, 80138 Napoli (IT)
(74) Representative: Pace Napoleone, Maria
(86) International application number: PCT/IB2021/060750
(87) International publication number: WO 2022/112912

(56) References cited:
- CN-U- 208 274 580
- US-A1- 2017 035 335
- US-A1- 2019 380 645

## Description

### TECHNICAL FIELD

The present invention relates to a portable vessel scanner. In particular, the present invention relates to a portable scanning device for improving the identification of a blood vessel of an individual at a skin region to be analyzed and promoting a blood sampling or the administration of an intravenous solution or drug.

### BACKGROUND ART

The identification of a blood vessel for performing sampling or an infusion of a substance into a patient is a complex operation which must necessarily be carried out by qualified personnel. However, identifying a vein or artery in the presence of a rather deteriorated or difficult vascular structure due to low patient blood pressure values (syncope or shock) can be a very complex undertaking even for competent personnel, especially if in situations of medical and/or home emergencies. Furthermore, the visualization of the vein and/or artery alone is not sufficient to facilitate sampling or cannulation. According to the guidelines of the Italian Society of Diagnostic and Therapeutic Medicine in association with the National Federation of Nursing Orders and Professions published in the "Journal of Nursing Infusion" (2019), the correct execution of venous, arterial and vascular cannulation depends on three fundamental conditions: the visualization of the vessel, the size of the vessel and the limitation of the movement of the vessel during the puncture of the wall.

In a hospital setting it is clearly possible to use various types of technologies to facilitate the detection of blood vessels. For example, so-called eco-guided venous access can be used for cannulation or sampling purposes which is very useful in the presence of patients with difficult vascular structure, for example in the case of adult and paediatric patients with peripheral venous plexus of the upper limbs which is difficult to find.

Ultrasound is a feasible, safe, effective and efficient technique in hospitals and emergency rooms which have an ultrasound instrument available in little time. However, in the case of a sampling and/or cannulation procedure for blood samplings or for infusion therapies, the identification of blood vessels can be difficult when the doctor is at the patient's home or if he is in public environments or in emergency rooms without ultrasound.

Therefore, an object of the present invention is to provide a vessel scanning device which partially or completely overcomes the drawbacks of the aforementioned known devices. In particular, it is an object of the present invention to provide a portable device which is structurally simple, light and not bulky while maintaining high levels of efficiency and reliability. An exemplary prior art device is disclosed in the patent publication US 2017/035335 A1.

### SUMMARY OF DISCLOSURE

These objects are achieved by a scanner according to the claims at the end of the present description.

The device according to the present disclosure mainly serves to improve the identification of a blood vessel of an individual at a skin region to be analyzed and to promote a blood sampling or the administration of a solution or drug intravenously and comprises at least one ultrasound probe to identify the position of the blood vessel by receiving and transmitting ultrasonic signals and acquiring data, a heating unit to generate heat at the blood vessel in the skin region to be analyzed, a stabilization mechanism to maintain the blood vessel at the position identified by the ultrasound probe, a control unit connected to the ultrasound probe, the heating unit and the stabilization mechanism to control the reception and transmission of said ultrasonic signals and analyze the acquired data, to regulate the generation of heat and to manage the position of the blood vessel, a display unit connected to the control unit to view the analyzed data and a support element in contact with at least the ultrasound probe, the heating unit, the stabilization mechanism, the control unit or the display unit and fixable to the individual's body to hold at least the ultrasound probe firmly in contact with said skin region.

By defining that the support element is in contact with at least one of the above-mentioned components (the probe, the heating unit, the stabilization mechanism, the control unit or the display unit), it is meant that the support element is configured to engage, attach or fix with at least one of these components so as to define a support structure for all the components which can thus be securely fixed to the patient's body.

Through this device, it is possible to correctly locate a patient's blood vessels even in complex situations when the patient's vascular structure is impaired and adequate instruments are not available, such as an ultrasound to perform an ultrasound-guided venous access. In particular, this device allows to perform any type of vascular procedure, such as a venous/arterial sampling or an intravenous therapy even at the patient's home. Furthermore, unlike the portable and/or hand-held ultrasounds already on the market, there is greater ease of execution and feasibility of the vascular procedure. In fact, unlike the standard eco-guided samplings which include the use of at least two operators (the first which identifies the vessel with the probe and the second which proceeds with the vascular procedure), the device according to the present invention can be used by a single operator who identifies the vessel with the ultrasound probe and subsequently fixes the device through the support, for example to the patient's arm so as to be able to freely perform the vascular procedure, increases the vascular surface through the heat generated by the heating unit, blocks the vessel by means of the stabilization mechanism and therefore significantly facilitates the vascular procedure.

Moreover, unlike the current portable and/or hand-held ultrasounds, the device according to the present invention has a relatively low production cost. In fact, this device is essentially dedicated only to vascular evaluation and therefore requires a system for identifying and fixing the small blood vessel essentially comprising a small ultrasound probe, a small heating element and a mechanical stabilization mechanism, for example roughly corresponding to the size of the display unit.

In addition, with respect to instruments which use wireless ultrasound probes, the device according to the present invention is more reliable as it avoids the risk of data loss or distortion during the transfer from the probe to the data analysis and information display unit. Furthermore, the present device is a compact system and does not require separate additional components, for example, to display the obtained data.

The device according to the present invention can be used by all medical or paramedical personnel even without specific ultrasound skills. Furthermore, the device can be used at the patient's home and in all operating conditions without ultrasound support or personnel competent in ultrasounds.

According to an embodiment, the ultrasound probe can comprise a plurality of receiver and transmitter transducers arranged contiguously to determine a linear scan.

In particular, each transducer of the ultrasound probe can consist of at least one piezoelectric crystal operating at a frequency of 4MHz.

According to an embodiment of the device, the control unit can comprise an electronic board and a microprocessor to generate electrical control signals to be sent to the ultrasound probe, the heating unit and/or the stabilization mechanism.

Furthermore, the control unit can comprise at least one memory to store the acquired and analyzed data.

According to an embodiment, the device can further comprise an ultrasound gel dispenser positioned at the ultrasound probe. Thereby, simply actuating a dedicated actuator, the gel is released into an area between the skin region to be analyzed and the ultrasound probe so as to increase the conductivity of the ultrasonic signals. The actuator can be mechanically actuated by the operator, for example by pushing a button, or electrically actuated by a control unit command, for example automatically after a time interval following the switching on of the device. It should be noted that the dispenser is provided with a tank to hold a sufficient amount of gel for at least a single application. The device further comprises a port for inserting new gel into the tank, once the latter is depleted.

According to an embodiment of the device, the display unit can comprise a liquid crystal display, LCD. To increase the compactness of the device, the screen can be small, for example similar to a watch face.

In particular, the display unit, i.e., the LCD screen, can be connected to the control unit via a flexible flat cable.

According to an embodiment of the device, the support element can comprise an adjustable strap or tie. Thereby, the device can be fixed to the body of a patient, for example to a limb of the individual, finding the most suitable position and easily identifying the blood vessel via the display unit. By virtue of the adjustable strap, it is also possible to keep the ultrasound probe in position and firmly in contact (directly or indirectly by means of an interposed medium, for example a gel) with the skin region to be analyzed.

Furthermore, the support element can comprise a protective structure for at least partially containing the ultrasound probe (and the dispenser, if present), the heating unit, the stabilization mechanism, the control unit and the display unit and in which said protective structure comprises at least one opening to allow the contact of the ultrasound probe, the heating unit and/or the stabilization mechanism with the skin region to be analyzed. For example, this protective structure can comprise a frame element surrounding the display unit and a rear surface in contact with the patient's body having the above-mentioned opening at the ultrasound probe, the heating unit, and/or the stabilization mechanism. Advantageously, an opening can have a longitudinal shape having dimensions corresponding to the length of the ultrasound probe which can be linear. Thereby, the ultrasound probe, although contained and protected within the protective structure, is in contact (direct or indirect) with the skin region to be analyzed. Similarly, an opening can have a different shape (circular, transverse) to reflect the dimensions of the heating unit and/or the stabilization mechanism.

According to an embodiment, the heating unit can comprise a heatable resistive element which can be, for example, battery powered. Furthermore, the heating unit can comprise an actuator, in particular a button, to activate the heating unit for a certain time, for example for the time necessary for the correct dilation of the blood vessel. This time can range from 10 to 15 seconds.

In particular, the heating unit can be connected to a microprocessor to automatically regulate the generation of heat so that the change in temperature of the skin region to be analyzed does not exceed a change in threshold temperature, in particular does not exceed 5°C.

In a further embodiment, the stabilization mechanism can comprise at least one shaft element with two cams, in which the shaft element is rotatable in steps defined by a crown element for the exit of the cams from the stabilization mechanism. Thereby, a slight pressure can be exerted on the sides of the blood vessel.

In particular, the two cams can be arranged at a distance between 0.8 cm and 1.2 cm, preferably 1.0 cm.

Furthermore, the shaft element can comprise a spring release mechanism for the re-entry of the cams into the stabilization mechanism upon the completion of the blood vessel stabilization process.

These and other aspects of the present invention will become more apparent by reading the following description of some preferred embodiments described below.
- Fig. 1: shows a block depiction of the device.
- Fig. 2a-b: show a schematic depiction of the device with front view (a) and rear view (b).
- Fig. 3: shows a block depiction of some components of the device.

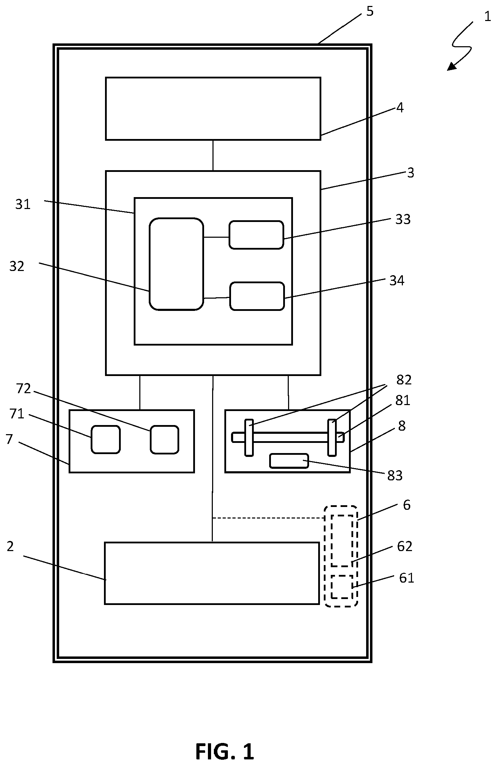

Figure 1 shows a block diagram of a schematic depiction of the portable vessel scanning device 1 to identify the presence and position of a blood vessel of an individual at a skin region to be analyzed. The device 1 consists of an ultrasound probe 2 and a control unit 3 connected to the probe 2. In particular, the control unit 3 comprises an electronic board 31 provided with a microprocessor 32 capable of generating control signals in sequence and acquiring the electrical signals corresponding to the echoes reflected by the tissues of the individual analyzed. The device 1 further comprises a heating unit 7 connected to the control unit 3. The heating unit 7 comprises a resistive heating element 71 and a button 72 to activate the heating element 71. The heating element 71 can be powered by a dedicated battery or by the battery 34 also used to power other components of the device 1. Additionally, the device comprises a stabilization mechanism 8. Such a mechanism comprises a shaft element 81 having two cams 82 capable of being rotated, for example by a crown, to allow the movement of the cams 82 out of and into the mechanism 8. When the cams 82 are inside the mechanism 8, they are not in contact with the skin region to be analyzed. When they are instead (at least partially) outside the mechanism 8, the cams 82 are in direct contact with the skin region to be analyzed and in particular serve to keep the blood vessel in place. The stabilization mechanism 8 further comprises a spring release mechanism 83 for the re-entry of the cams 82 inside the mechanism. The device 1 comprises a display unit 4 for receiving the data acquired and analyzed by the control unit 3 and displaying it, for example, on a liquid crystal display. Lastly, the device 1 comprises a support 5 which is in contact with at least one of the components defined above (e.g., at least the probe 2, the heating unit 7, the stabilization mechanism 8, the control unit 3 and the display unit 4 and which can be fixed to the body of an individual. In particular, the support 5 can be configured to at least partially contain one of the components defined above.

Furthermore, the control unit 3 comprises a memory 33 to store the acquired signals and transfer them by means of a serial interface to the display unit 4 and a battery 34 to power the various components of the device 1. The battery 34 can be any replaceable battery or an integrated rechargeable battery via a dedicated external power supply. Alternatively, the battery can be recharged via an alternative power generation system, for example a photovoltaic component positioned on an external surface of the device 1.

The device 1 can additionally comprise a gel dispenser 6 positioned at the ultrasound probe 2. The dispenser 6 is depicted with a dashed mark, as it is optional and can be absent from the device 1. In fact, the gel can be applied to the skin region to be analyzed by the operator himself if necessary through a separate external dispenser.

If the dispenser 6 is present, the device 1 comprises an actuator 61 to actuate the dispenser 6 and release gel in an area between the skin region to be analyzed and the ultrasound probe 2. The actuator 61 can be operated mechanically by the operator, for example by pressing a button, or electrically by a control unit 3 command, in particular by the microprocessor 32. The dispenser 6 is provided with a tank 62 to contain an amount of gel sufficient for at least a single application.

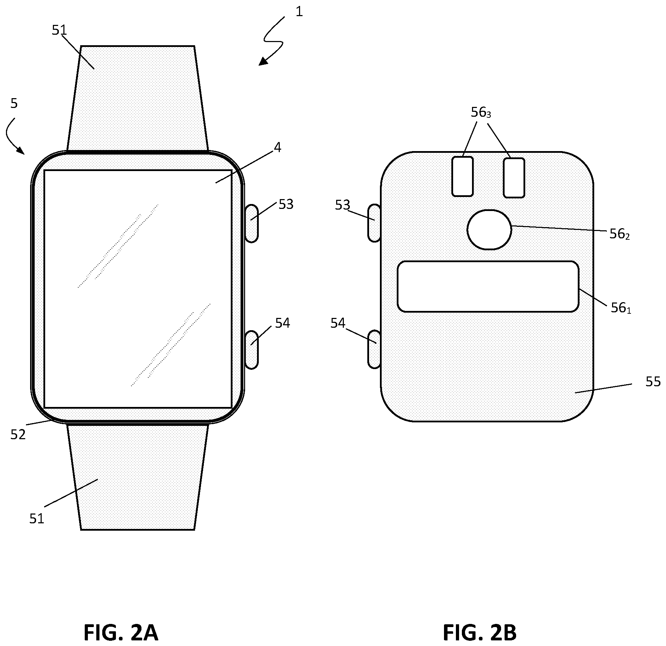

Figure 2A shows the device 1 seen from the front. The support 5 of the device 1 comprises an adjustable strap 51 to fix the device 1 to the body of a patient (for example to the arm) and a protective structure 52 to contain and at least partially protect the other components of the device 1. According to this configuration, it should be noted that the device itself takes the form of a wristwatch, in which the adjustable strap 51 represents the bracelet, the protective structure 52 represents the case and the bezel while the display unit 4 represents the dial of the watch. The device 1 further comprises an on button 53 and an off button 54 positioned on one side of the protective structure 52. The display unit 4 is rectangle-shaped. However, it is evident that this unit 4 can assume other shapes (e.g., square, circular, oval, etc.).

Figure 2B shows the device 1 seen from behind. For visual convenience, the adjustable strap 51 was omitted from figure 2B. It should be noted in the figure that the protective structure 52 comprises a rear surface 55 which is positioned in direct contact with the patient's body. The rear surface 55 is provided with various openings 56₁, 56₂, 56₃. For example, there is a longitudinal opening 56₁ at the ultrasound probe 2 (not shown in the figure) to allow (direct or indirect) contact between the probe 2 and the skin region to be analyzed. Furthermore, another circular opening 56₂ is present at the heating unit 7 (not shown in the figure) to allow contact (direct or indirect) between the heating unit 7 and the skin region to be analyzed. In addition, there are two vertical openings 56₃ at the stabilization mechanism 8, in particular at the cams 82 (not shown in the figure) to allow the (direct or indirect) contact between the cams 82 and the skin region to be analyzed.

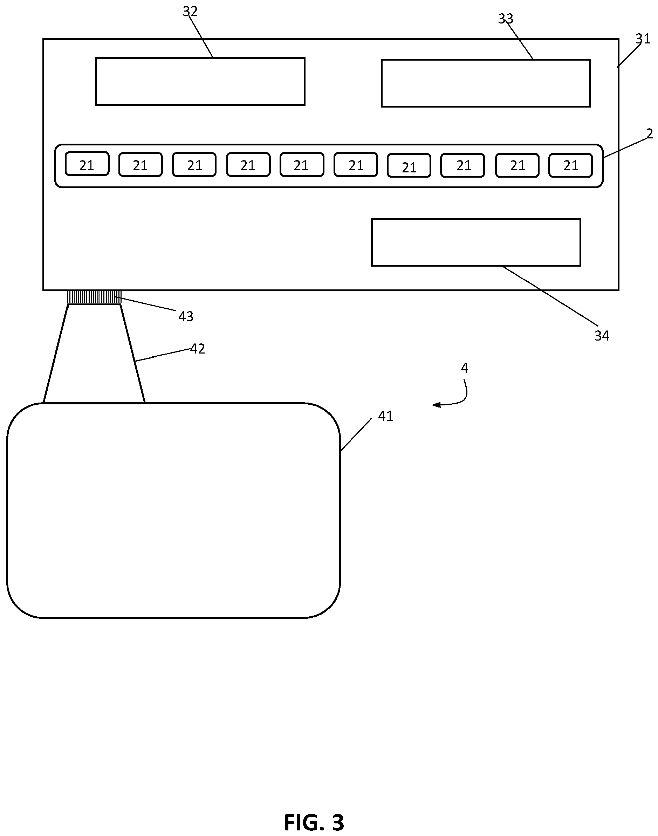

Figure 3 schematically shows some components of the device 1. The ultrasound probe 2 is a linear probe with an approximate length of 10 cm and consists of an array of transmitting/receiving piezoelectric transducers 21. The figure shows a group of ten contiguously placed transducers 21 excited in succession by the control unit 3 so as to form a linear scan. In particular, the array of transducers 21 is welded onto an electronic board 31 capable of driving the individual elements to generate ultrasonic pulses lasting less than a microsecond. On the board 31 there is a microprocessor 32 capable of generating the control signals in sequence and acquiring the electrical signals corresponding to the echoes reflected by the skin tissues investigated. In this case, the resolution in depth is less than a millimetre for a distance of about 40 mm. The acquired signals are stored in a memory bank 33 and subsequently transferred to a screen 41, for example a grey-tone LCD display of the size of about 50mm x 30mm for viewing. The display 41 is connected to the electronic board 31 via a flexible flat cable 42 and connector 43.

With the device 1 described herein, it is possible to facilitate the correct execution of three fundamental steps for correct sampling and correct cannulation, i.e., the visualization of the vessel, by means of an ultrasound probe 2, the greater visibility of the blood vessel by increasing the size of the vessel by means of the vasodilation induced by a heat source of the heating unit 7, and the limitation of the movement of the vessel during the puncture of the wall, i.e., the "clamping" of the vessel by means of an inverted U-shaped stabilization mechanism 8 (determined by the cams 82 which exit from the mechanism 8) which limits the lateral movements of the vessel wall. Therefore, the rear part of the device 1 comprises a small ultrasound probe 2 which offers the possibility to view the vessel, a resistive heating element 71 which delivers heat locally and finally a mechanical stabilization mechanism 8 which can be operated by means of a camshaft and a crown.

Thereby, it is possible to perform a correct vascular sampling or therapy procedure with a small and portable support which can be used in any situation. Specifically, once the vessel is visualized with the ultrasound probe 2, the feasibility of the sampling is evaluated based on the diameter of the vessel. In the presence of small vessels (paediatric patients), sclerotic vessels (patients undergoing repeated procedures, elderly, neoplastic, etc.) the heating element 71 located on the vessel, identified by the ultrasound probe 2, determining a localized vasodilation favours the execution of the sampling by increasing the vascular diameter and therefore the vascular surface on which to operate. It has been seen that a temperature increase of 3-4°C, for a duration of 10-15 seconds, causes a vasodilation of about 40-60%.

Once the vascular surface with the heat source is increased, the vessel stabilization mechanism 8 limits the lateral movement of the vessel itself and thus facilitates the vascular puncture and correct insertion of the needle/cannula into the vascular bed. It is known that vascular wall puncture can cause vascular contraction and dislocation, as well as voluntary or involuntary movements of the patient, similarly, it can dislocate the vessel and compromise correct sampling execution. Therefore, the vascular stabilization mechanism 8, by blocking the vessel in the position displayed by the ultrasound probe 2, allows the sampling to be performed correctly even in the presence of contractions and movements of the patient.

A person skilled in the art can perform several and further modifications and variants to the device described above, in order to satisfy further and contingent needs, all said modifications and variants however included within the scope of protection of the present invention as defined by the appended claims.

## Claims

1. Portable vessel scanning device (1) for improving the identification of a blood vessel of an individual at a skin region to be analyzed, and facilitating blood sampling or intravenous administration of a solution or of a drug, the device (1) comprising:
- an ultrasound probe (2) to identify the position of blood vessel by receiving and transmitting ultrasonic signals and acquiring data;
- a heating unit (7) to generate heat at the blood vessel in the skin region to be analyzed;
- a stabilization mechanism (8) to maintain the blood vessel at the position identified by the ultrasound probe (2);
- a control unit (3) connected to the ultrasound probe (2), to the heating unit (7) and to the stabilization mechanism (8) to control the reception and transmission of said ultrasonic signals and analyze the acquired data, to regulate heat generation and to manage the position of the blood vessel;
- a display unit (4) connected to the control unit (3) to view the acquired and analyzed data, and
- a support element (5) in contact at least with the ultrasound probe (2), the heating unit (7) and the stabilization mechanism (8), the control unit (3), or the display unit (4) and fixable to the individual's body to hold the ultrasound probe (2) firmly in contact with said skin region,
**characterized in that**
the device (1) comprises an ultrasound gel dispenser (6) positioned at the ultrasound probe (2), the ultrasound gel dispenser (6) being provided with a tank, the device further comprising a port for inserting gel into the tank, and
the support element (5) comprises a protective structure (52) for at least partially containing the ultrasound probe (2), the ultrasound gel dispenser (6), the heating unit (7), the stabilization mechanism (8), the control unit (3) and the display unit (4), and wherein said protective structure (52) comprises a plurality of openings (56₁, 56₂, 56₃) to allow the contact of the ultrasound probe (2), of the heating unit (7) and of the stabilization mechanism (8), with the skin region to be analyzed, the plurality of openings comprising a longitudinal opening (56ᵢ) at the ultrasound probe (2), a circular opening (56₂) at the heating unit (7), and vertical openings (56₃) at the stabilization mechanism (8), the plurality of openings (56₁, 56₂, 56₃) being provided in a rear surface (55) of said protective structure (52) and being positioned at the ultrasound probe (2), at the heating unit (7), and/or at the stabilization mechanism (8).

2. Device (1) according to claim 1, wherein the ultrasound probe (2) comprises a plurality of receiving and transmitting transducers (21) arranged contiguously to determine a linear scan.

3. Device (1) according to claim 2, wherein each transducer (21) of the ultrasound probe (2) consists of at least one piezoelectric crystal operating at the frequency of 4MHz.

4. Device (1) according to one of the preceding claims, wherein the control unit (3) comprises an electronic board (31) and a microprocessor (32) to generate electrical control signals to be sent to the ultrasound probe (2), to the heating unit (7) and/or to the stabilization mechanism (8).

5. Device (1) according to one of the preceding claims, wherein the control unit (3) comprises at least one memory (33) to store the acquired and analyzed data.

6. Device (1) according to one of the preceding claims, wherein the display unit (4) comprises a liquid crystal screen (41).

7. Device (1) according to one of the preceding claims, wherein the display unit (4) is connected to the control unit (3) via a flexible flat cable (42).

8. Device (1) according to one of the preceding claims, wherein the support element (5) comprises an adjustable strap (51).

9. Device (1) according to one of the preceding claims, wherein the the heating unit (7) comprises:
a. a resistive heating element (71); and/or
b. an actuator (72), namely a button, to activate the heating element (7) for a given time.

10. Device (1) according to one of the preceding claims, wherein the the heating unit (7) is connected to a microprocessor (32) to automatically regulate the generation of heat so that the change in temperature of the skin region to be analyzed does not exceed a change in threshold temperature, in particular does not exceed 5°C.

11. Device (1) according to one of the preceding claims, wherein the stabilization mechanism (8) comprises at least a shaft element (81) having two cams (82), and wherein the shaft element (81) is capable of being rotated at defined steps by means of a crown shaped element to allow the movement of the cams (82) out of the stabilization mechanism (8).

12. Device (1) according to claim 11, wherein two cams (82) are arranged at a distance comprised between 0.8 cm and 1.2 cm, preferably 1.0 cm.

13. Device (1) according to claim 11 or 12, wherein the shaft element (81) further comprises a spring release mechanism (83) for the re-entry of the cams (82) inside the stabilization mechanism (8).

## Patentansprüche

1. Tragbares Gefäßabtastgerät (1) zum Verbessern der Identifikation eines Blutgefäßes eines Individuums in einem zu analysierenden Hautbereich und Ermöglichen von Blutentnahme oder intravenöser Verabreichung einer Lösung oder einer Arznei, wobei das Gerät Folgendes umfasst:
- eine Ultraschallsonde (2) zum Identifizieren der Position des Blutgefäßes durch Aufnehmen und Übermitteln von Ultraschallsignalen und Erfassen von Daten;
- ein Heizgerät (7) zum Erzeugen von Wärme an dem Blutgefäß in dem zu analysierenden Hautbereich;
- einen Stabilisierungsmechanismus (8) zum Halten des Blutgefässes an der durch die Ultraschallsonde (2) identifizierten Position;
- ein Steuergerät (3), das an die Ultraschallsonde (2), an das Heizgerät (7) und an den Stabilisierungsmechanismus (8) zum Steuern der Aufnahme und Übermittlung der Ultraschallsignale und Analysieren der erfassten Daten angeschlossen ist, um die Wärmeerzeugung zu regulieren und die Position des Blutgefäßes zu handhaben;
- ein Anzeigegerät (4), das an das Steuergerät (3) zum Betrachten der erfassten und analysierten Daten angeschlossen ist und
- ein Stützelement (5) in Kontakt mit mindestens der Ultraschallsonde (2), dem Heizgerät (7) und dem Stabilisierungsmechanismus (8), dem Steuergerät (3) oder dem Anzeigegerät (4) und das an den Körper des Individuums fixierbar ist, um die Ultraschallsonde (2) fest in Kontakt mit dem Hautbereich zu halten,
**dadurch gekennzeichnet, dass**
das Gerät (1) einen Ultraschallgelverteiler (6) umfasst, der an der Ultraschallsonde (2) positioniert ist, wobei der Ultraschallgelverteiler (6) mit einem Tank ausgestattet ist, wobei das Gerät ferner eine Öffnung zum Einführen von Gel in den Tank umfasst und
das Stützelement (5) ein Schützgefüge (52) umfasst zum mindestens teilweisen Enthalten der Ultraschallsonde (2), des Ultraschallgelverteilers (6), des Heizgeräts (7), des Stabilisierungsmechanismus (8), des Steuergeräts (3) und des Anzeigegeräts (4) und wobei das Schützgefüge (52) eine Mehrzahl von Öffnungen (56₁, 56₂, 56₃) umfasst, um den Kontakt der Ultraschallsonde (2), des Heizgeräts (7) und des Stabilisierungsmechanismus (8) mit dem zu analysierenden Hautbereich zu gestatten, wobei die Mehrzahl von Öffnungen ein Längsöffnung (56₁) an der Ultraschallsonde (2), eine runde Öffnung (56₂) am Heizgerät (7) und eine senkrechte Öffnung (56₃) am Stabilisierungsmechanismus (8) umfasst, wobei die Mehrzahl von Öffnungen (56₁, 56₂, 56₃) in einer hinteren Fläche (55) des Schützgefüges (52) bereitgestellt und an der Ultraschallsonde (2), am Heizgerät (7) und/oder am Stabilisierungsmechanismus (8) positioniert ist.

2. Gerät (1) nach Anspruch 1, wobei die Ultraschallsonde (2) eine Mehrzahl von aufnehmenden und übermittelnden Messwandlern (21) umfasst, die zum Bestimmen einer linearen Abtastung zusammenhängend angeordnet sind.

3. Gerät (1) nach Anspruch 2, wobei jeder Messwandler (21) der Ultraschallsonde (2) aus mindestens einem piezoelektrischen Kristall besteht, das mit der Frequenz von 4 HMz funktioniert.

4. Gerät (1) nach einem der vorhergehenden Ansprüche, wobei das Steuergerät (3) eine elektronische Leiterplatte (31) und einen Mikroprozessor (32) zum Erzeugen elektrischer Steuersignale umfasst, die an die Ultraschallsonde (2), an das Heizgerät (7) und/oder an den Stabilisierungsmechanismus (8) geschickt werden sollen.

5. Gerät (1) nach einem der vorhergehenden Ansprüche, wobei das Steuergerät (3) mindestens ein Speicher (33) zum Speichern der erfassten und analysierten Daten umfasst.

6. Gerät (1) nach einem der vorhergehenden Ansprüche, wobei das Anzeigegerät (4) einen Flüssigkristallbildschirm (41) umfasst.

7. Gerät (1) nach einem der vorhergehenden Ansprüche, wobei das Anzeigegerät (4) an das Steuergerät (3) über ein flexibles flaches Kabel (42) angeschlossen ist.

8. Gerät (1) nach einem der vorhergehenden Ansprüche, wobei das Stützelement (5) ein einstellbares Band (51) umfasst.

9. Gerät (1) nach einem der vorhergehenden Ansprüche, wobei das Heizgerät (7) Folgendes umfasst:
a. ein widerstandsfähiges Heizelement (71); und/oder
b. einen Auslöser (72), nämlich einen Druckknopf zum Aktivieren des Heizelements (7) für eine vorgegebene Zeit.

10. Gerät (1) nach einem der vorhergehenden Ansprüche, wobei das Heizgerät (7) an einen Mikroprozessor (32) zum automatischen Regulieren der Erzeugung von Wärme angeschlossen ist, so dass die Temperaturänderung des zu analysierenden Hautbereichs eine Änderung der Schwellentemperatur nicht übersteigt, insbesondere 5 °C nicht übersteigt.

11. Gerät (1) nach einem der vorhergehenden Ansprüche, wobei der Stabilisierungsmechanismus (8) mindestens ein Wellenelement (81) umfasst, das zwei Nocken (82) aufweist und wobei das Wellenelement (81) in der Lage ist, in definierten Schritten durch ein kronenförmiges Element rotiert zu werden, um die Bewegung der Nocken (82) aus dem Stabilisierungsmechanismus (8) zu gestatten.

12. Gerät (1) nach Anspruch 11, wobei zwei Nocken (82) in einer Entfernung angeordnet sind, die zwischen 0,8 cm und 1,2 cm, bevorzugt 1,0 cm, liegt.

13. Gerät (1) nach Anspruch 11 oder 12, wobei das Wellenelement (81) ferner einen Federfreigabemechanismus (83) zum Wiedereintreten der Nocken (82) in den Stabilisierungsmechanismus (8) umfasst.

## Revendications

1. Dispositif portable de balayage des vaisseaux sanguins (1) destiné à améliorer l'identification d'un vaisseau sanguin d'un individu au niveau d'une zone cutanée devant être analysée, et à faciliter le prélèvement sanguin ou l'administration intraveineuse d'une solution ou d'un médicament, le dispositif (1) comprenant :
- une sonde à ultrasons (2) destinée à identifier la position du vaisseau sanguin en recevant et en émettant des signaux ultrasonores et en acquérant des données ;
- une unité de chauffage (7) destinée à générer de la chaleur au niveau du vaisseau sanguin dans la zone cutanée devant être analysée ;
- un mécanisme de stabilisation (8) destiné à maintenir le vaisseau sanguin à la position identifiée par la sonde à ultrasons (2) ;
- une unité de commande (3) reliée à la sonde à ultrasons (2), à l'unité de chauffage (7) et au mécanisme de stabilisation (8) destinée à commander la réception et l'émission desdits signaux ultrasonores et à analyser les données acquises, à réguler la production de chaleur et à gérer la position du vaisseau sanguin ;
- une unité d'affichage (4) reliée à l'unité de commande (3) destinée à visualiser les données acquises et analysées, et
- un élément de support (5) en contact au moins avec la sonde à ultrasons (2), l'unité de chauffage (7) et le mécanisme de stabilisation (8), l'unité de commande (3) ou l'unité d'affichage (4), et pouvant être fixé au corps du sujet afin de maintenir la sonde à ultrasons (2) fermement en contact avec ladite région cutanée,
**caractérisé en ce que**
le dispositif (1) comprend un distributeur de gel à ultrasons (6) positionné au niveau de la sonde à ultrasons (2), le distributeur de gel à ultrasons (6) étant muni d'un réservoir, le dispositif comprenant en outre un orifice permettant d'introduire du gel dans le réservoir, et
l'élément de support (5) comprend une structure de protection (52) destinée à contenir au moins partiellement la sonde à ultrasons (2), le distributeur de gel à ultrasons (6), l'unité de chauffage (7), le mécanisme de stabilisation (8), l'unité de commande (3) et l'unité d'affichage (4), et dans lequel ladite structure de protection (52) comprend une pluralité d'ouvertures (56₁, 56₂, 56₃) pour permettre le contact de la sonde à ultrasons (2), de l'unité de chauffage (7) et du mécanisme de stabilisation (8) avec la zone cutanée à analyser, la pluralité d'ouvertures comprenant une ouverture longitudinale (56i) au niveau de la sonde à ultrasons (2), une ouverture circulaire (56₂) au niveau de l'unité de chauffage (7), et des ouvertures verticales (56₃) au niveau du mécanisme de stabilisation (8), la pluralité d'ouvertures (56₁, 56₂, 56₃) étant prévues dans une surface arrière (55) de ladite structure de protection (52) et étant positionnées au niveau de la sonde à ultrasons (2), de l'unité de chauffage (7) et/ou du mécanisme de stabilisation (8).

2. Dispositif (1) selon la revendication 1, dans lequel la sonde à ultrasons (2) comprend une pluralité de transducteurs de réception et d'émission (21) disposés de manière contiguë afin de réaliser un balayage linéaire.

3. Dispositif (1) selon la revendication 2, dans lequel chaque transducteur (21) de la sonde à ultrasons (2) est constitué d'au moins un cristal piézoélectrique fonctionnant à une fréquence de 4 MHz.

4. Dispositif (1) selon l'une des revendications précédentes, dans lequel l'unité de commande (3) comprend une carte électronique (31) et un microprocesseur (32) destinés à générer des signaux de commande électriques à envoyer à la sonde à ultrasons (2), à l'unité de chauffage (7) et/ou au mécanisme de stabilisation (8).

5. Dispositif (1) selon l'une des revendications précédentes, dans lequel l'unité de commande (3) comprend au moins une mémoire (33) destinée à stocker les données acquises et analysées.

6. Dispositif (1) selon l'une des revendications précédentes, dans lequel l'unité d'affichage (4) comprend un écran à cristaux liquides (41).

7. Dispositif (1) selon l'une des revendications précédentes, dans lequel l'unité d'affichage (4) est reliée à l'unité de commande (3) par l'intermédiaire d'un câble plat flexible (42).

8. Dispositif (1) selon l'une des revendications précédentes, dans lequel l'élément de support (5) comprend une sangle réglable (51).

9. Dispositif (1) selon l'une des revendications précédentes, dans lequel l'unité de chauffage (7) comprend :
a. un élément chauffant résistif (71) ; et/ou
b. un actionneur (72), à savoir un bouton, destiné à activer l'élément chauffant (7) pendant une durée donnée.

10. Dispositif (1) selon l'une des revendications précédentes, dans lequel l'unité (7) est reliée à un microprocesseur (32) afin de réguler automatiquement la production de chaleur de telle sorte que la variation de température de la zone cutanée à analyser ne dépasse pas un seuil de température, en particulier ne dépasse pas 5 °C.

11. Dispositif (1) selon l'une des revendications précédentes, dans lequel le mécanisme de stabilisation (8) comprend au moins un élément d'arbre (81) comportant deux cames (82), et dans lequel l'élément d'arbre (81) peut être mis en rotation par paliers définis au moyen d'un élément en forme de couronne afin de permettre le déplacement des cames (82) hors du mécanisme de stabilisation (8).

12. Dispositif (1) selon la revendication 11, dans lequel les deux cames (82) sont espacées d'une distance comprise entre 0,8 cm et 1,2 cm, de préférence 1,0 cm.

13. Dispositif (1) selon la revendication 11 ou 12, dans lequel l'élément d'arbre (81) comprend en outre un mécanisme de libération à ressort (83) pour le retour des cames (82) à l'intérieur du mécanisme de stabilisation (8).
